# EUROPÄISCHE PATENTSCHRIFT

(11) **EP 0 583 265 B1**
(45) Veröffentlichungstag und Bekanntmachung des Hinweises auf die Patenterteilung: **23.09.1998**
(21) Anmeldenummer: 92908537.1
(22) Anmeldetag: 14.04.1992
(51) Int. Cl.: C12Q 1/68, C12N 15/10, C12P 21/00

(54) **VERFAHREN ZUR HERSTELLUNG VON NEUEN BIOPOLYMEREN**
METHOD FOR PREPARING NEW BIOPOLYMERS
PROCEDE DE PREPARATIONS DE NOUVEAUX BIOPOLYMERES

(30) Priorität: 16.04.1991 DE 4112440
(43) Veröffentlichungstag der Anmeldung: 23.02.1994
(73) Patentinhaber: Evotec BioSystems GmbH, D-22529 Hamburg (DE)
(72) Erfinder: HENCO, Karsten, D-4006 Erkrath 2 (DE); EIGEN, Manfred, D-3400 Göttingen (DE)
(74) Vertreter: Meyers, Hans-Wilhelm, Dr.Dipl.-Chem.
(86) Internationale Anmeldenummer: EP9200840
(87) Internationale Veröffentlichungsnummer: WO9218645

(56) Entgegenhaltungen:
- EP-A- 285 123
- WO-A-91/05058
- WO-A-92/02536
- SCIENCE. Bd. 249, 3. August 1990, LANCASTER, PA US Seiten 505 - 510; C.TUERK ET AL.: 'Systematic evolution of ligands by exponential enrichment: RNA ligands to bacteriophage T4 DNA polymerase' in der Anmeldung erwähnt
- BERICHTE DER BUNSEN-GESELLSCHAFT FüR PHYSIKALISCHE CHEMIE Bd. 89, 1985, WEINHEIM,DE Seiten 658 - 667; M.EIGEN: 'Macromolecular evolution: dynamical ordering in sequence space'
- NATURE. Bd. 344, 29. März 1990, LONDON GB Seiten 467 - 468; D.L.ROBERTSON ET AL.: 'Selection in vitro of an RNA enzyme that specifically cleaves single-stranded DNA'
- GENE. Bd. 82, 1989, AMSTERDAM NL Seiten 83 - 87; G.F.JOYCE: 'Amplification,mutation and selection of catalytic RNA'

## Beschreibung

Gegenstand der vorliegenden Erfindung ist ein Verfahren zur Herstellung von neuen Biopolymeren mit verbesserten Eigenschaften mittels Polymerasen. Biopolymere wie Peptide und RNA werden stets synthetisiert unter Mitwirkung von Polymerasen. Um neue Biopolymere mit verbesserten Eigenschaften zu erhalten, hat man in der Vergangenheit vor allem nach neuen biologischen Quellen gesucht und diese selektiert, um schließlich solche mit guten oder besseren Eigenschaften im großen Umfang zu replizieren und dadurch die gewünschten Biopolymere zu produzieren. Letztlich beruhen hierauf alle klassischen biologischen und mikrobiologischen Züchtungsmethoden.

Durch die Technik der Rekombination von Genen und der Produktion von Biopolymeren in Mikroorganismen und Zellkulturen konnten bereits erhebliche Fortschritte erzielt werden. Versuche durch gezielte oder ungezielte Veränderung einzelner oder mehrerer Teile der Biopolymeren bzw. der kodierenden Nukleinsäuresequenzen haben trotz des erheblichen Aufwandes nur in wenigen Fällen zum Erfolg geführt. L. Gold beschreibt die Optimierung einer RNA über eine oder zwei gleichwertige Punktmutationen innerhalb eines engen Sequenzausschnittes nach einem Verfahren, das SELEX genannt wurde. Die Natur zeigt jedoch, daß häufig entferntere Sequenzabschnitte oder längere zusammenhängende Sequenzabschnitte zum Gegenstand gezielter Optimierung werden. Beispiele hierfür sind die Optimierung von Antikörpern nach Mechanismen, die als somatische Mutationen beschrieben sind. Somatische Mutationen sind mechanistisch noch nicht vollends verstanden, zeigen jedoch eine bedeutende Steigerung der Selektivität eines Antikörpers und erweitern somit das genetische Potential kodierter Antikörper über ihre Fähigkeit zur variierenden Rekombination hinaus.

Die Optimierung biologischer Pharmaprodukte ist ein wichtiges Ziel der pharmazeutischen Industrie. Erwähnt seien die Optimierung von enzymatischen Aktivitäten, von Rezeptoreigenschaften, von Ligandenwechselwirkungen, von antibiotischen oder antiviralen Eigenschaften, von effektiver Vaccinierung etc. Technisch werden bislang viele Wege verfolgt wie Screeningmethoden auf bereits natürlich vorhandene Strukturen oder deren chemische Modifizierung, wie zielorientiertes Design durch Computermodelling oder rekombinant gentechnische Verfahren.

Die Natur offenbart uns die Ergebnisse eines natürlichen Optimierungsprozesses, d. h., die Ergebnisse der Evolution. Ihre Mechanismen zu verstehen, war das Anliegen vieler namhafter Naturwissenschaftler und Philosophen. Es soll hier in wenigen Sätzen versucht werden, die Auffassungen Darwins ("The origin of Species", G. G. Simpson, Collier-Mac Millan, London, 1962), Monods ("Zufall und Notwendigkeit", J. Monod, Piper, München, 1971) und Eigens (M. Eigen, J. McCaskill & P. Schuster, 1987, 1988, J. Phys. Chem.) kurz zu analysieren. Darwin kam bei seinem Studium der Arten zu dem Ergebnis, daß Evolution auf dem Wechselspiel von Mutation und natürlicher Selektion beruht. Monod arbeitete diese Gedanken in das neue Weltbild der molekularen Genetik ein und diskutierte die Bedeutung zufälliger Mutationen der genetischen Information eines existierenden Wildtyps und ihre zwangsläufige Selektion im Falle einer besser angepaßten Wertigkeit.

Eigen konnte anhand einfacher Überlegungen demonstrieren, daß der natürliche Optimierungsprozess der Evolution durch Wildtyp-Mutagenese nicht erklärbar ist. Anders ausgedrückt, ein blindes Ausprobieren natürlich entstehender Wildtypmutanten könnte angesichts unvorstellbar großer Zahlen möglicher Alternativen niemals zu einer effizienten Evolution führen. Der abgelaufene Zeitraum von ca. 4 Milliarden Jahren wäre zu kurz, die zur Verfügung stehende Materie um viele Größenordnungen zu klein, um auch nur zu primitiven Lebensformen zu gelangen. Der Durchbruch im Verständnis evolutiver Vorgänge gelang durch die richtige Interpretation genetischer Experimente sowie durch die Einführung neuer Konzepte, wie Quasi-Spezies, Sequenzraum, Wertlandschaft, Hyperzyklus, Kompartimentierung.

Für die vorliegende Erfindung sind Erklärungen zu einigen Begriffen unbedingt notwendig, da sie für die beanspruchte erfinderische Umsetzung in wirtschaftlich nutzbare Synthesestrategien unumgänglich sind.

"Quasi-Spezies" bezeichnet ein neues Verständnis der alten "Wildtyp"-Definition. Die Gesamtheit einer durch Reduplikation auseinander hervorgegangenen Kollektion von Genomen stellt keine Sammlung identischer (Wildtyp)-Sequenzen dar. Vielmehr handelt es sich um eine Verteilung von Mutanten in der Umgebung des Wildtyps. Die Verteilung als solche ist das Objekt der Selektion, nicht die spezifische "Wildtyp"-Sequenz. Die Verteilung entsteht vor allem durch Kopierfehler reduplikativer Enzyme (Polymerasen). Lediglich die häufigst vertretene mittlere Sequenz ist identisch mit dem zuvor angenommenen "Wildtyp", während die große Mehrheit aller übrigen Sequenzen mehr oder weniger starke Abweichungen zur ehemals definierten Wildtyp-Sequenz aufweisen. Die Häufigkeit einer spezifischen Sequenz und die Häufigkeit eng verwandter Sequenzen stellt ein Maß für die biologische Wertigkeit dar. Die Verteilung bestimmt die sogenannte Wertlandschaft. Der evolutive Vorteil liegt unmittelbar auf der Hand: Bei verändertem Anforderungsprofil kann ein so strukturiertes biologisches System rasch reagieren. Wenn auch zunächst zahlenmäßig nicht stark vertreten, so gibt es doch Varianten, die relativ weit entfernt vom Wildtyp, jedoch näher am hypothetisch neuen Maximum einer Wertigkeit liegen. Die Evolution erhält dadurch eine interne Steuerung, und es wird Zeit gewonnen. Dem positiven Effekt entgegen steht die Gefahr des Informationsverlustes durch zu große Replikationsfehlerhäufigkeit. Eigen konnte zeigen, daß natürliche Systeme dicht unterhalb des zulässigen Fehlermaximums replizieren. Ein irreversibles Zerfließen genetischer Information wird verhindert, doch ist die Flexibilität für Veränderungen maximal. Die Quasi-Spezies bleibt stabil, solange sich keine besser angepaßte Verteilung aufbaut. Eine weitere Folgerung dieses neuen Verständnisses von Wertlandschaften ist es, daß blinde "trial and error" Mutageneseprozesse nicht erforderlich sind, sondern daß sich evolutive Anpassungen zielgerichtet entlang von "Graten" der Wertlandschaft bewegen. Hyperzyklus und Kompartimentierung sind weitere Begriffe, die erfinderisch in den Technikmaßstab umgesetzt werden und in ihrer Natur kurz erklärt werden sollen. Der Hyperzyklus stellt die zyklische Interaktion von replikationsfähigen Informationsträgern und den durch sie kodierten Replikation-katalysierenden Enzymen dar. Die Kompartimentierung in Vesikel oder Zellstrukturen sorgt dafür, daß Varianten von Hyperzyklen für sich evoluieren können und daß verhindert wird, daß sich Mutationen statistisch und gleichmäßig auswirken, und somit nicht direkt und spezifisch auf die kodierende Sequenz rückwirken.

Die Erfindung hat sich die Aufgabe gestellt, unter Ausnutzung obiger Erkenntnisse ein Verfahren zur Herstellung von neuen Biopolymeren mit systematisch verbesserten Eigenschaften mittels Polymerasen zu entwickeln. Diese Aufgabe kann gelöst werden dadurch, daß mindestens ein Zyklus der nachstehend genannten Schritte in einer Serie von zu vergleichenden Parallelansätzen durchlaufen wird,
- Nukleinsäuresequenzen oder ein Gemisch ähnlicher Nukleinsäuresequenzen in der Mutantenverteilung einer Quasi-Spezies werden im Bereich der Fehlerschwelle einer begrenzten Mutagenese unterworfen,
- diese Gemische werden unter simultanen Bedingungen und/oder nacheinander repliziert,
- die so entstandenen Nukleinsäuregemische werden durch Aufteilung kompartimentiert
- und danach durch ein Selektionssystem selektiert, welches die interessierenden Eigenschaften der Nukleinsäuresequenz selbst oder indirekt über deren Translationsprodukt reflektiert.

Die Mutagenese erfolgt im einfachsten Fall bereits durch die Replikation im Bereich der Fehlerschwelle des Replikationsenzyms/Enzymsystems. Darüber hinaus kann sie auch erfolgen durch Reaktionsbedingungen und Reaktionsparameter, die Mutationen begünstigen, um in den Bereich der zulässigen Fehlerschwelle für ein Gensegment zu gelangen oder kurz darüber hinaus. Hierzu zählen beispielsweise die definiert ungleichgewichtige Gabe von Purin- und/oder Pyrimidinnukleotidbasen sowie die Zugabe von Basenanalogen. Weiterhin können auch mutagene Substanzen und/oder energiereiche Strahlung angewendet werden. Schließlich können auch sonstige Parameter wie Temperatur, pH-Wert, Redoxpotential etc. Mutationen begünstigen.

Unter begrenzter Mutation im Sinne der vorliegenden Erfindung ist eine Mutagenese zu verstehen, die noch nicht zur völligen Zerstörung des Systems führt. Sie beabsichtigt weiterhin, nicht sämtliche Teile der Nukleinsäuresequenz zu verändern. Dies bedeutet jedoch nicht, daß für Teile des Gemisches und kurzzeitig doch die biologisch zulässige Grenze überschritten wird. Es ist also durchaus zulässig, daß neben überlebensfähigen und gegebenenfalls optimierten Mutanten auch geschädigte Mutanten entstehen, die letztlich nicht überleben. Auf alle Fälle soll nicht nur eine einzele Mutante überleben, sondern es müssen Gruppen von Mutanten überleben. Die Bedingungen der Mutagenese können darüber hinaus durchaus so gewählt oder abgestuft werden, daß unterschiedliche Nukleinsäuresequenzabschnitte unterschiedlichem Mutagenesedruck ausgesetzt sind. Aber auch bei dieser Ausführungsform ist darauf zu achten, daß Gruppen von Mutanten überleben.

Vorzugsweise erfolgt die Mutagenese so, daß durch die Einstellung der als Variablen benutzten Genauigkeit der Replikation oder deren abgestufte Genauigkeit in konsekutiven Schritten um den Bereich der Fehlerschwelle herum ("Tempern"), gefolgt von der (>> 10) genaueren Verstärkungsreplikation die Verteilung der Nukleinsäuren auf das gesuchte Optimum hin gerichtet durch die Wertlandschaft im Bereich ihrer Fehlerschwelle geführt wird.

Die Aufteilung in Kompartimente muß erfindungsgemäß so erfolgen, daß einerseits praktisch alle neuen und alle alten Varianten in mindestens einem Kompartiment vorhanden sind, andererseits der Inhalt der Kompartimente durch phänotypische Selektion differenziert werden kann. Diese Bedingungen können von Fall zu Fall durch relativ einfache Vorversuche ermittelt werden. Als Kompartimente sind beispielsweise geeignet die Kompartimente in den Replikationsmaschinen nach Eigen; vgl. Patentanmeldungen DE-U-88 13 773.2 ("Verschiebeeinheit"), DE-U-88 14 398.8 ("Gradientenbrett"), DE 40 22 792.8 ("Folie"), DE 40 22 793.6 ("Verschweißeinrichtung"), DE 40 22 794.4 ("Form d. Folie") und DE 40 29 004.2 ("Schlitten").

Für die praktische Durchführung des Verfahrens ist es weiterhin wichtig, daß die zu einer phänotypisch selektierten Biopolymerengruppe zugehörigen Nukleinsäuresequenzen simultan oder nachträglich allein oder im Gemisch zugeordnet werden können. Dies ist vor allem deshalb nötig, um die erfindungsgemäß ermittelten optimierten Nukleinsäuresequenzen wiederzufinden und gezielt für weitere Zyklen einsetzen zu können.

Im Gegensatz zu den klassischen Methoden der Gentechnologie werden erfindungsgemäß weder die genauen Nukleinsäuresequenzen der Ausgangspopulation noch die genauen Nukleinsäuresequenzen der optimierten Population bestimmt. Es wird vielmehr ausgehend von den Variationsbreiten ausgewählter Gruppen durch Mutagenese und Selektionierung weiterentwickelt. Hierdurch werden die Gratwanderungen der Natur bei der Evolution ausgenutzt und dadurch das Gesamtverfahren beschleunigt und vereinfacht.

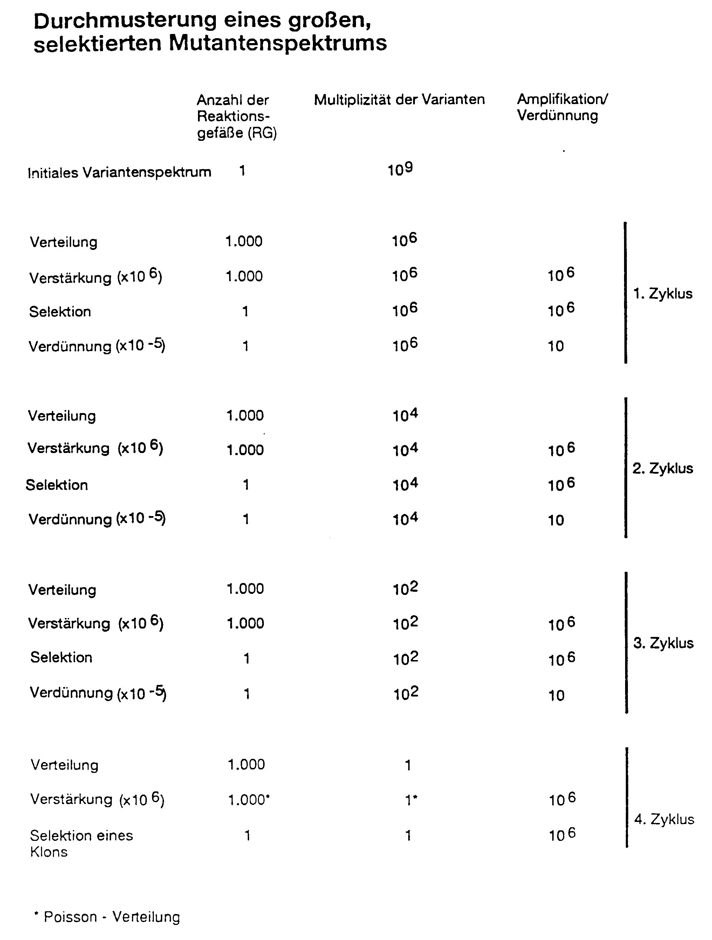

Die Selektionierung der Nukleinsäuregemische erfolgt vorzugsweise an Produkten, welche direkte Kopplungen von Genotypen und Phänotypen darstellen. Ein Beispiel hierfür sind Polysome, die in einer direkten Kopplung sowohl die Translationsprodukte als auch deren kodierende RNA enthalten. Gruppen derartiger Polysome können beispielsweise biologisch relevant üblicherweise selektiert werden zum Beispiel durch ihre Eigenschaft der Bindung an bestimmte Rezeptoren, wie Antikörper, Metallchelatkomplexe etc. An diesen Polysomen wiederum ist die relevante Nukleinsäuresequenz noch angekoppelt. Sie kann daher in besonders einfacher Weise nachträglich wieder zugeordnet werden. Selbstverständlich sind aber auch andere übliche Methoden der Selektionierung möglich. Beispielsweise kann eine derartige Durchmusterung eines großen selektierten Mutantenspektrums auch auf den Apparaturen nach Eigen erfolgen. Sie ermöglicht aus einem initialen Variantenspektrum von 10⁹ in bereits vier Zyklen dieser Apparatur die Selektion eines einzigen Klons. Die zahlenmäßigen Bereiche, in denen diese Selektionierung sinnvoll und effizient durchführbar sind, ergeben sich aus der anliegenden Figur 1 über die hierarchische Parallelführung und Selektion sowie die zugehörigen Zahlenbeispiele für die Durchmusterung eines großen selektierten Mutantenspektrums. Als besonders geeignete Verfahren zur Detektion haben sich hierbei Fluoreszenznachweisverfahren erwiesen.

Wenn eine Mutantenverteilung bemerkenswerte Eigenschaften aufweist, läßt sich durch hierarchische Parallelführung ein großes Spekturm von Mutanten durchmustern. Für das Beispiel einer Multiplizität von 10⁹ Varianten ist man bereits nach 4 Selektionszyklen bei der Stufe einer Reinkultur.

Um statistisch mit hoher Wahrscheinlichkeit keine Varianten zu verlieren, werden nach Verstärkung so viele Mutanten in den nächsten Zyklus überführt, daß jede der selektionierten Varianten ca. 10 x vertreten ist. Bei Zahlenwerten << 10 würde der stochastische Effekt maßgebend, so daß leicht Variantenverluste eintreten können. Umgekehrt würde die durchschnittliche Repräsentanz von 100 Kopien jeder Variante keinen Selektionseffekt bzw. Reduktion der Variantenanzahl bewirken.

Bei der Durchführung des erfindungsgemäßen Verfahrens sollte deshalb darauf geachtet werden, daß nach Möglichkeit die folgenden Parameter erfüllt werden:
- Zyklisch geführte Reaktionskette mit von außen steuerbarer biologischer Funktionskontrolle und Selektion;
- reproduzierbare Handhabung großer Probenmengen;
- möglicher Verzicht auf rekombinante Organismen;
- zyklische, künstlich induzierte Verbreiterung des Mutantensprektrums mit regioselektiver Mutagenese;
- On-Line-Kontrolle der Optimierung;
- Möglichkeit zur Generierung statistischer Verteilungen großer Quasi-Spezies-Populationen;
- Optimierung der Population entlang von Graten der Wertlandschaft;
- hierarchische Parallelführung vieler Probenansätze.

Hiermit unterscheidet sich das erfindungsgemäße Verfahren deutlich von bisher bekannten Verfahren. Die Idee, aus einem Kollektiv randomisiert angebotener Mutanten gut angepaßte Substrukturen zu selektionieren, ist bereits von Rechenberg 1973 vorgeschlagen worden ("Evolutionsstrategie", Problemata frommannholzboog, Stuttgart-Bad Cannstatt). C. Tuerk und L. Gold (1990), Science 249, 505 - 510, konnten experimentell auch zeigen, daß diese Vorgehensweise zum Erfolg führt, wenn die Anzahl möglicher Mutanten klein ist, d. h. die Anzahl der zu variierenden Positionen gering ist und somit alle möglichen Varianten a priori in einem Gemisch repräsentiert sind. Das bedeutet jedoch in einem Kolletiv von angenommen 10¹² Molekülen, ca. 18 Positionen auf DNA/RNA-Ebene, wenn jede mögliche Sequenz mit durchschnittlich 10 Molekülen besetzt sein soll. Mit dieser Strategie lassen sich nur äußerst kurze Bereiche optimieren. Ziel einer Selektion sollen jedoch erfindungsgemäß Proteine sein, an denen große Teile der Sequenzen optimiert werden können und die dennoch als Gruppe von Mutantenverteilungen vorliegen. Mit einer a priori Verteilung müßte eine unvorstellbar große Anzahl von Molekülen bearbeitet werden. Dies kann aus den verschiedenstens Gründen, insbesondere aus den Gründen des unverhältnismäßig großen Aufwandes nicht funktionieren. Es würden wiederum alle Dimensionen an Material und benötigter Zeit gesprengt.

Auch die Verfahren des sogenannten "serial transfer" sind zumindestens in der bisher in der Literatur beschriebenen Form nicht praktikabel, wenn der Selektionsschritt und die Bewertung der spezifischen Mutante eine Verdünnung auf Einzelkopieebene verlangt. Damit lassen sich technisch zu wenig Mutanten testen und werten. Erfindungsgemäß wird von dem Prinzip der Natur Gebrauch gemacht, in welcher die besser angepaßten Mutanten weder einem homogenen Sequenzspektrum noch einer definierten Wildtypsequenz entsprechen. Die Natur arbeitet hingegen mit Mutantenverteilungen und einer Wichtung der Quasi-Spezies. Dieses Prinzip ist bisher nie zur gezielten Weiterentwicklung und Wertigkeitsbestimmung herangezogen worden. Es sind deshalb auch nicht die sie umgebene Wertlandschaft des Sequenzraumes inklusive solcher Mutanten mit großen Hamming-Abständen berücksichtigt worden. Gerade die Population von Mutanten in weiterer Entfernung zur häufigst besetzten Sequenz und deren engsten Umgebungen vermehrt zu besetzen, wird erfindungsgemäß ausgenutzt. Dabei wird die Besetzung der Wertlandschaft der Quasi-Spezies-Verteilung verbreitert, d. h. geschmolzen. Unter diesem Schmelzen wird verstanden, das begrenzte Überschreiten zulässiger Fehlergrenzen. Die zulässige Fehlergrenze (Einbauraten von nicht "Watson-Crick-Paaren" ist definiert durch die Länge der Sequenz und den Selektionswert einer Quasi-Spezies-Verteilung, d. h. der Höhenverteilung der Werteprofile. Da die Fehleinbauraten der verwendeten Polymerasen bezogen auf die in vitro replizierten Sequenzen gewöhnlich klein sind, wird der Mutationsdruck erfindungsgemäß künstlich erhöht.

Abgestimmt auf den Mutationsdruck muß dann die Stringenz des Selektionssystems gewählt werden. Die Stringenz muß so groß sein, daß das Profil der Besetzung der Wertlandschaft folgt und bestehen bleibt im Gegensatz zur statistischen Gleichverteilung beim SELEX-Verfahren. Die Bedingungen sind somit auch so zu wählen, daß Mutanten mit großen relativen Hamming-Abständen die Selektion überstehen können.

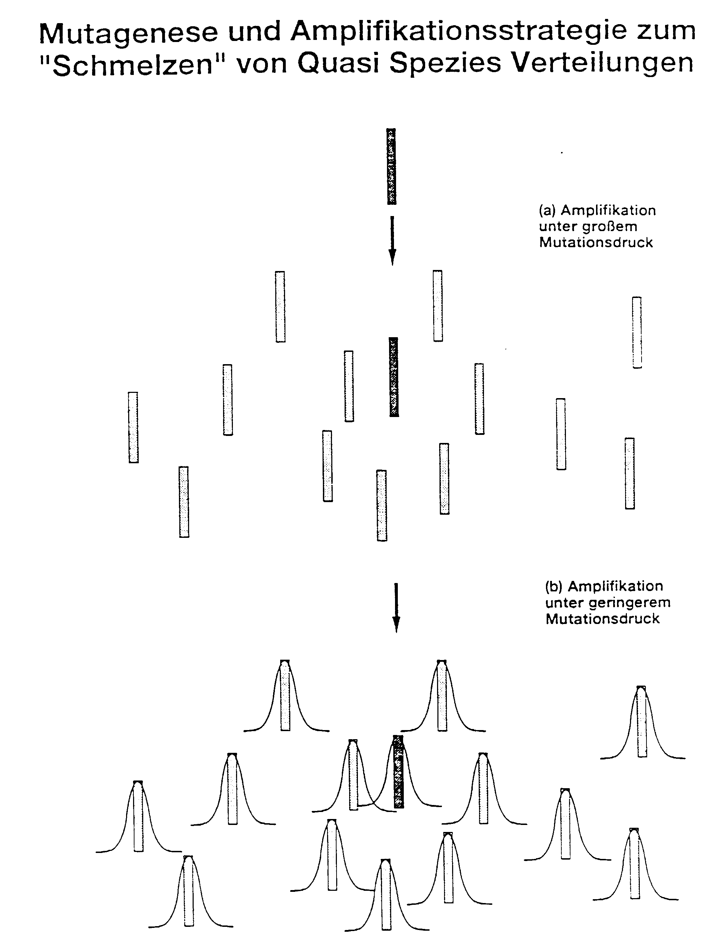

Das erfindungsgemäße Konzept der Quasi-Spezies berücksichtigt auch die Besetzungsdichte sogenannter neutraler Mutanten. Im engsten Sinne neutral sind nur die Mutanten, die auch in der Nachbarschaft von gleichwertigen Mutanten umgeben sind, d. h. einem Plateau in der Wertlandschaft. Dies kann jedoch nur annähernd gelten. Der Wert einer spezifischen Sequenz wird mitbestimmt von der Bewertung und somit Besetzung der benachbarten Varianten, aus denen eben diese Mutante bei Replikation und geringer Mutagenese hervorgehen kann. Erfindungsgemäß sollten deshalb die Bedingungen so gewählt werden, daß eine hinreichende Besetzungsdichte der Wertlandschaft erhalten bleibt. Es wird daher erfindungsgemäß unterschieden zwischen dem Mutageneseschritt zur Schaffung von Varianten großer Hamming-Abstände und dem Replikationsschritt zur Besetzung der jeweiligen engeren Hamming-Abstände in der Umgebung der weit entfernten Varianten. Dieses Prinzip wird augenscheinlich in der anliegenden Darstellung der Mutagenese und Amplikationsstrategie zum Schmelzen von Quasi-Spezies-Verteilungen; Figur 2.

Diese stilisierte Darstellung beschreibt das Schicksal einer Mutantensequenz aus einer Mutantenverteilung, die den vorhergehnden Selektions- und Verteilungsschritt überstanden hat. Im Mutagenese-Schritt wird diese Sequenz in einem "Mutagenese-Puls" geschmolzen, d. h. daß Mutanten entstehen, die einen höheren mittleren Hamming-Abstand von den Ausgangsmutanten haben, als es der natürlichen Fehleinbaurate der beteiligten Polymerasen entsprechen würde. Um eine funktionale Selektion betreiben zu können, folgt dem Mutageneseschritt erfindungsgemäß ein Verstärkungsschritt (Amplifikation). Hierbei wird der Mutationsdruck experimentell deutlich niedriger gewählt, im Extremfall entsprechend der natürlichen Fehleinbaurate der verwendeten Polymerasen. Damit ergibt sich um die mittlere Sequenz der weit voneinander divergierenden Varianten jeweils ein Mutantenspektrum mit kleineren Hamming-Abständen.

Verfahren, die sich für die besprochene Vorgehensweise eignen, sind automatisierte Genamplifikationsverfahren, die zyklisch oder alternierend zyklisch gefahren werden und das Prozessieren umfangreicher Kollektive erlauben. Dafür wurden PCR-taugliche Systeme entwickelt und/oder serial Transfer-Systeme, wobei umfangreiche Quasi-Spezies-Verteilungen transferiert und selektiert werden können. Bei der erfindungsgemäßen Umsetzung der Technik kommt es, wie oben beschrieben, auf die technische Beherrschung großer Probenkollektive an und deren Wichtung mittels geeigneter Selektionssysteme. Andere Amplifikationsverfahren (z. B. J. Compton 1991, Nature 350, 91 - 92) sind unter bestimmten Bedingungen ebenfalls für den Verstärkungs- und Mutageneseschritt geeignet.

Mit der beschriebenen Folientechnik zur Genamplifikationsstrategie lassen sich technisch zwischen 10³ und 10⁶ Reaktionsansätze parallel bewerten. Das bedeutet letztlich die Analyse und Selektion aus bis zu 10¹³ bis 10¹⁶ Mutanten pro Reaktionszyklus.

Das erfindungsgemäße Wechselspiel von Selektionsdruck und Mutationsdruck sorgt für die Balance zwischen Zerfließen des Informationsgehaltes in ein Kontinuum zahlenmäßig nicht mehr zu bewältigender Besetzungsmöglichkeiten einerseits und Erstarren des Systems andererseits. Es ist fein abgestimmt, um ein optimales Driften der Quasi-Spezies hin zum gesuchten Optimum zu gestatten. Das Verfahren der Natur konnte zwar analysiert werden und gibt zahlenmäßig wertvolle Hinweise für die größenordnungsmäßige Wahl der experimentellen Parameter, das Feintuning wird jedoch erfindungsgemäß jeweils angepaßt, da es spezifisch mit dem jeweiligen Anforderungsprofil korreliert ist und sich im Verlaufe des Optimierungsprofils verändern kann. Mit anderen Worten, beide Parameter sind immer wieder einander anzugleichen. Sie spiegeln die jeweilige Struktur der Wertlandschaft wieder, d. h. die Zerklüftung oder die Breite der Grate und Bergrücken. Kleine Fehlabweichungen eines Parameters können durch Nachführen des zweiten Parameters nachgesteuert werden, so daß die Gefahr beherrscht werden kann, daß z. B. in einem fortgeschrittenen Optimierungsstadium durch zu hohen Mutationsdruck die Information irreversibel zerfließt. Konkret bedeutet dies, daß bei zu niedrig gewähltem Mutationsdruck der Selektionsdruck erhöht werden kann und bei zu hoch gewähltem Mutationsdruck der Selektionsdruck verringert werden kann.

Von großer Bedeutung für die Adjustierung der diskutierten Parameter ist die Wahl des Selektionsverfahrens, mit dem es möglich sein muß, kontrolliert Quasi-Spezies-Verteilung zu selektionieren. Die Parameter sollen die Wertigkeit einer Population korrekt messen können, d. h. sie sollen mit dem angestrebten Optimum eng korreliert sein. Ihr Signal/Rausch-Verhältnis muß hinreichend groß sein, um bei der Parallelführung von Experimenten im automatisierten Vielkanalsystem klare Entscheidungen zuzulassen. Fluoreszenzmeßverfahren eignen sich optimal für diesen Zweck.

Vorzugsweise sollen Selektionsdrucke ausgeschlossen werden, die nicht im direkten Zusammenhang mit dem Ziel optimum stehen. So wird die Replikationsgeschwindigkeit als Kriterium ausgeschlossen, indem amplifizierende Enzyme, Primer im Überschuß angeboten werden. Bei Einsatz der in vitro Proteinbiosynthese lassen sich Effekte ausschließen, die auf Gebrauch seltener Codons zurückzuführen sind.

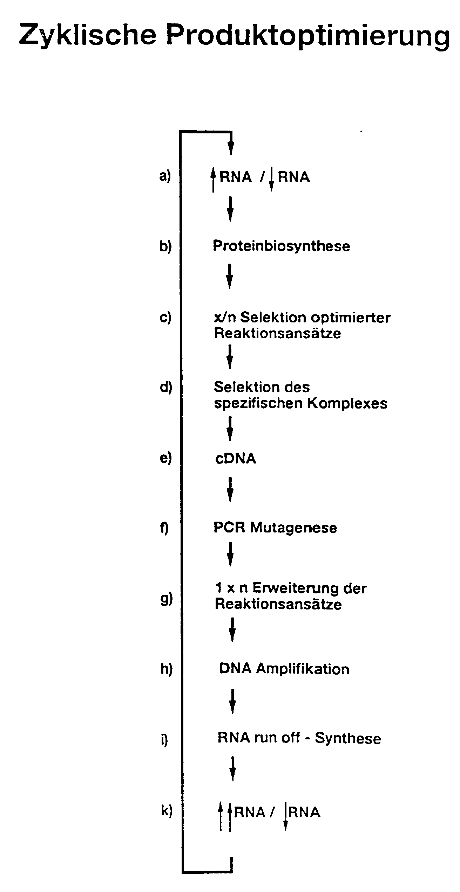

Erfindungsgemäß wird der Zyklus mindestens einmal, vorzugsweise jedoch mehrfach durchgeführt. In Figur 3 ist eine typische zyklische Produktoptimierung gezeigt. Sie kann beispielsweise die Schritte a) bis k) beinhalten. Dabei wird eine zu optimierende, informationstragende RNA entweder mittels Replikase oder in einer PCR-Reaktion oder einem homogenen RNA/DNA-Replifikationsvefahren mutagenisiert und repliziert. Dabei wird für eine Übersetzung in das zugehörige Proteinprodukt eine Promotor-Sequenz eingeführt, die sich z. B. über primer oder Ligationsverfahren synthetisch einführen läßt und somit dem Mutationsschritt nicht unterworfen wird. Zur Kopplung von Genotyp und Phänotyp lassen sich Bindungseigenschaften mit gezielter Extraktion verbinden, wodurch der Selektionsschritt eines Zyklus in seiner Effizienz gesteigert werden kann. Erfindungsgemäß notwendig und ausreichend ist jedoch bereits die Kompartimentierungsstrategie zur hierarchischen Parallelführung von Mutantenverteilungen.

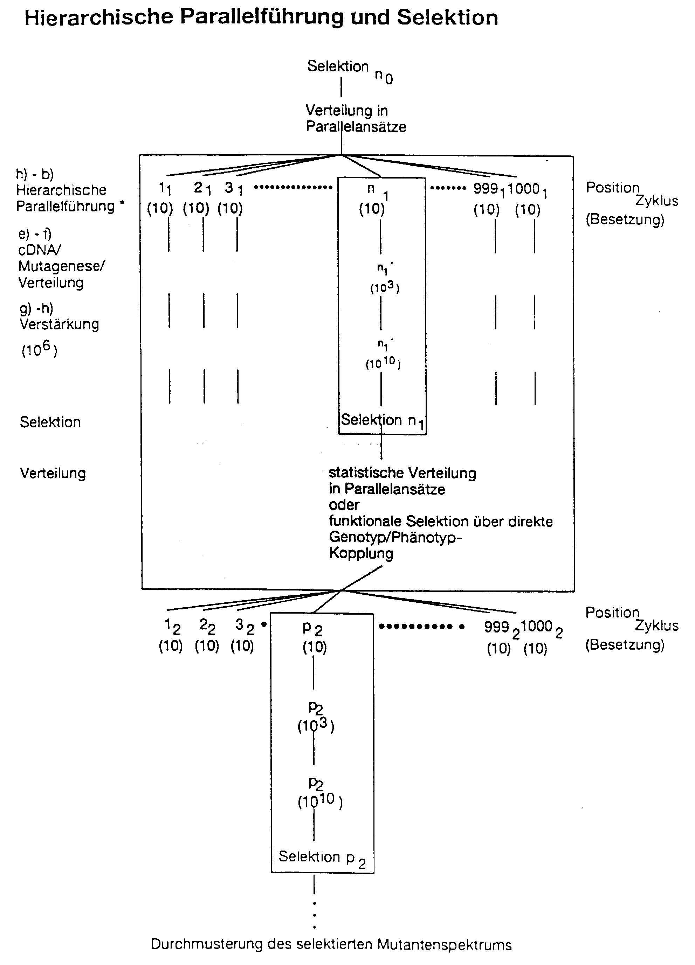

Figur 4 zeigt eine typische hierarchische Parallelführung nach Selektion. Das Diagramm verdeutlicht einen Ausschnitt aus der Kette zyklischer Reaktionsabfolgen, mit der die erfindungsgemäße hierarchische Parallelführung und der Bezug zu Mutations-, Amplifikations- und Selektionsschritt verdeutlicht wird. Die angegebenene Zahlen entsprechen dem erfindungsgemäßen Verfahren. Sie können real jedoch erheblich abweichen. Ihre Größenordnungen werden jeweils so gewählt, daß
- optimierte Varianten eine statistisch hohe Chance haben, positiv selektioniert zu werden bzw. den jeweils nächsten Zyklus zu erreichen,
- optimierte Varianten sich im Untergrundrauschen minderwertiger Varianten im Selektionsschritt zu erkennen geben,
- die Amplifikationshöhe ein hinreichend großes Meßsignal des Selektionsparameters erlaubt,
- möglichst viele unterschiedliche Varianten pro Zyklus durchgemustert werden.

Ein Aliquot einer selektierten Variantenverteilung m des Zyklus o (mo) mit ca 1.000 Varianten wird so auf viele (z. B. 1.000) Reaktionsgefäße (RG) verteilt, daß jedes RG ca. 10 Varianten enthält, also jede Variante insgesamt ca. 10 x repräsentiert ist. Somit ist die Wahrscheinlichkeit des Verlustes einer Variante statistisch klein. Die Verteilungen des ersten Zyklus (1₁, 2₁, ... 1.000₁) werden parallel der Mutagenese unterworfen, wobei ca. 10³ Varianten pro RG entstehen. Sie werden so verstärkt, daß insgesamt ca. 10¹⁰ Sequenzen pro RG entstehen, die die 10³ unterschiedlichen Varianten nach Mutagenese beinhalten.

Erfindungsgemäß wird der Reaktionsansatz n₁ im Selektionsschritt für den nächsten Zyklus ausgewählt, jedoch nicht über die Bewertung von n₁, sondern über die Bewertung des von n₁ abgeleiteten, verstärkten Mutantenspektrums n₁'. Für das Beispiel von 10³ Varianten pro RG reicht es wieder, wenn ein Aliquot so auf die 1.000 RG des Folgezyklus verteilt wird (1₂, 2₂ ... 1.000₂), daß im Durchschnitt wieder jede einzelne Variante aus n₁' 10fach im Zyklus 2 repräsentiert wird. Auf der Basis der angenommenen Zahlenwerte würden pro Zyklus 10⁵ Varianten gemustert. Diese Zahl läßt sich deutlich steigern, wenn entweder mehr RG eingesetzt werden, ein sensitiver oder schärfer selektierendes Selektionsverfahren eingesetzt wird oder wenn erwünschte Mangelmutanten z. B. durch Extraktion von n₁' abgetrennt werden können, wie es z. B. bei einer Selektion über Bindungsoptimierung möglich ist.

## Patentansprüche

1. Verfahren zur Herstellung von neuen Biopolymeren mit verbesserten Eigenschaften mittels Polymerasen, dadurch gekennzeichnet, daß mindestens ein Zyklus der nachstehend genannten Schritte in einer Serie von zu vergleichenden Parallelansätzen durchlaufen wird,
a) Nukleinsäuresequenzen oder ein Gemisch ähnlicher Nukleinsäuresequenzen in der Mutantenverteilung einer Quasi-Spezies werden im Bereich der Fehlerschwelle für ein Gensegment einer begrenzten Mutagenese unterworfen,
b) diese Gemische werden unter simultanen Bedingungen und/oder nacheinander repliziert,
c) die so entstandenen Nukleinsäuregemische werden durch Aufteilung kompartimentiert, wobei pro Zyklus die Schritte a) bis c) mindestens einmal durchlaufen werden,
und danach durch ein Selektionssystem selektiert, welches die interessierenden Eigenschaften der Nukleinsäuresequenz selbst oder indirekt über deren Translationsprodukt reflektiert.

2. Verfahren nach Anspruch 1, dadurch gekennzeichnet, daß die begrenzte Mutagenese durch definiert ungleichgewichtige Gabe der Purin- und/oder Pyrimidinnukleotidbasen oder Basenanaloge oder durch mutagene Substanzen und/oder durch energiereiche Strahlung oder sonstige die Mutation begünstigende Reaktionsparameter erzeugt wird.

3. Verfahren nach einem der Ansprüche 1 bis 2, dadurch gekennzeichnet, daß bei der Mutagenese durch die Einstellung der als Variablen benutzten Genauigkeit der Replikation oder deren abgestufte Genauigkeit in konsekutiven Schritten um den Bereich der Fehlerschwelle eines Genabschnittes herum ("Tempern"), gefolgt von der (>> 10) genaueren Verstärkungsreplikation die Verteilung der Nukleinsäuren auf das gesuchte Optimum hin qerichtet durch die Wertlandschaft im Bereich ihrer Fehlerschwelle geführt wird.

4. Verfahren nach einem der Ansprüche 1 bis 3, dadurch gekennzeichnet, daß unterschiedliche Nukleinsäuresequenzabschnitte unterschiedlichem Mutagenesedruck ausgesetzt werden.

5. Verfahren gemäß einem der Ansprüche 1 bis 4, dadurch gekennzeichnet, daß die Aufteilung in Kompartimente so erfolgt, daß einerseits praktisch alle neuen und alten Varianten für sich in mindestens irgendeinem Kompartiment vorhanden sind und andererseits der Inhalt der Kompartimente durch phänotypische Selektion differenziert werden kann.

6. Verfahren gemäß Anspruch 5, dadurch gekennzeichnet, daß die zu einer phänotypisch selektierten Biopolymerengruppe zugehörigen Nukleinsäuresequenzen simultan oder nachträglich alleine oder im Gemisch zugeordnet werden können.

7. Verfahren nach mindestens einem der Ansprüche 1 bis 6, dadurch gekennzeichnet, daß die Selektionierung der Nukleinsäuregemische an Produkten erfolgt, welche direkte Kopplungen von Genotypen und Phänotypen darstellen.

8. Verfahren nach Anspruch 7, dadurch gekennzeichnet, daß die Selektionierung an Polysomen erfolgt, die in einer Kopplung sowohl die Translationsprodukte als auch deren kodierende RNA enthalten.

9. Verfahren nach einem der Ansprüche 1 bis 8, dadurch gekennzeichnet, daß die Selektion von Zyklus zu Zyklus durch Variation der Stringenz der Selektionsbedingungen und Mutationsbedingungen gesteuert wird.

10. Verfahren gemäß einem der Ansprüche 1 bis 9, dadurch gekennzeichnet, daß eine große Anzahl vergleichender Parallelansätze gefahren und ausgewertet wird.

## Claims

1. A process for the preparation of new biopolymers with improved properties using polymerases, characterized in that at least one cycle of the following steps is run in a series of parallel runs to be compared:
a) nucleic acid sequences or a mixture of similar nucleic acid sequences with the mutant distribution of a quasi-species are subjected to limited mutagenesis in the range of the error threshold for a gene segment;
b) said mixtures are replicated simultaneously and/or sequentially;
c) the mixtures of nucleic acids thus generated are compartmentalized by partition,
wherein steps a) to c) are run at least once per cycle, and then selected by a selection system which reflects the interesting properties of the nucleic acid sequence itself or indirectly through its translation product.

2. The process according to claim 1, characterized in that said limited mutagenesis is effected by a definedly unbalanced supply with purine and/or pyrimidine nucleotide bases or base analogues, or by mutagenic substances, and/or by high-energy radiation or other reaction parameters favoring mutation.

3. The process according to any of claims 1 to 2, characterized in that the distribution of the nucleic acids is directed through the value landscape in the range of its error threshold towards the sought optimum in said mutagenesis by adjusting the reliability of replication, used as a variable, or its gradual reliability in consecutive steps around the range of the error threshold for a gene segment ("tempering"), followed by a more reliable (by >> 10) amplification replication.

4. The process according to any of claims 1 to 3, characterized in that different segments of a nucleic acid sequence are subjected to different mutagenic pressures.

5. The process according to any of claims 1 to 4, characterized in that said partition into compartments is done in such a way that, on one hand, virtually all new and old variants by themselves are present in at least one compartment and, on the other hand, the contents of the compartments can be differentiated by phenotypical selection.

6. The process according to claim 5, characterized in that the nucleic acid sequences which belong to a phenotypically selected group of biopolymers can be assigned simultaneously or later, alone or in a mixture.

7. The process according to at least one of claims 1 to 6, characterized in that said selecting of the nucleic acid mixtures is done on products which represent a direct coupling of genotypes and phenotypes.

8. The process according to claim 7, characterized in that said selecting is done on polysomes which contain both the translation products and their coding RNAs coupled together.

9. The process according to any of claims 1 to 8, characterized in that said selecting is controlled from cycle to cycle by varying the stringency of the selection conditions and mutation conditions.

10. The process according to any of claims 1 to 9, characterized in that a large number of comparative parallel runs is performed and evaluated.

## Revendications

1. Procédé de préparation de nouveaux biopolymères présentant des propriétés améliorées, à l'aide de polymérases, caractérisé en ce qu'on parcourt au moins un cycle des étapes ci-après, selon une série d'opérations en parallèle à comparer,
a) des séquences d'acides nucléiques, ou un mélange de séquences d'acides nucléiques analogues, dans la distribution des mutants d'une quasi-espèce, sont soumis à une mutagenèse limitée dans le domaine du seuil d'erreur d'un segment génique,
b) ces mélanges sont répliqués, dans des conditions simultanées et/ou successivement,
c) les mélanges d'acides nucléiques ainsi produits sont compartimentés par répartition, les étapes a) à c) étant parcourues au moins une fois par cycle,
puis on les sélectionne grâce à un système de sélection qui reflète les propriétés intéressantes de la séquence d'acides nucléiques, directement, ou indirectement par l'intermédiaire de leur produit de traduction.

2. Procédé selon la revendication 1, caractérisé en ce que la mutagenèse limitée est produite par addition, non-équilibrée dans des conditions définies, des bases nucléotidiques puriques et/ou pyrimidiques, ou d'analogues de bases, ou grâce à des substances mutagènes, et/ou grâce à un rayonnement de haute énergie, ou d'autres paramètres de réaction qui favorisent la mutation.

3. Procédé selon l'une des revendications 1 à 2, caractérisé en ce que lors de la mutagenèse, grâce à l'ajustement de la précision, utilisée comme variable, de la réplication, ou de sa précision étagée dans le cadre d'étapes consécutives autour du domaine du seuil d'erreur d'un segment génique ("équilibrage"), suivies de la réplication par amplification, plus précise (>> 10), la répartition des acides nucléiques est guidée, en étant dirigée vers l'optimum recherché, à travers la région utile, dans le domaine de son seuil d'erreur.

4. Procédé selon l'une des revendications 1 à 3, caractérisé en ce que les différents segments de séquences d'acides nucléiques sont exposés à une pression de mutagenèse différente.

5. Procédé selon l'une des revendications 1 à 4, caractérisé en ce que la répartition en compartiments est réalisée de façon que d'une part la presque totalité des variants nouveaux et anciens soit présents en soi dans au moins un compartiment quelconque, et d'autre part le contenu des compartiments puisse être différencié par sélection phénotypique.

6. Procédé selon la revendication 5, caractérisé en ce que les séquences d'acides nucléiques appartenant à un groupe de biopolymères ayant subi une sélection phénotypique peuvent être affectés simultanément ou successivement, seuls ou en mélange.

7. Procédé selon au moins l'une des revendications 1 à 6, caractérisé en ce que la sélection des mélanges d'acides nucléiques s'effectue sur des produits qui représentent des couplages directs de génotypes et de phénotypes.

8. Procédé selon la revendication 7, caractérisé en ce que la sélection s'effectue sur des polysomes qui dans un couplage contiennent tant les produits de traduction que leur ARN codant.

9. Procédé selon l'une des revendications 1 à 8, caractérisé en ce que la sélection d'un cycle à l'autre est pilotée par une variation de la stringence des conditions de sélection et des conditions de mutation.

10. Procédé selon l'une des revendications 1 à 9, caractérisé en ce qu'un grand nombre d'opérations parallèles comparatives sont mises en oeuvre et évaluées.
